# EUROPEAN PATENT APPLICATION

(11) **EP 4 116 326 A1**
(43) Date of publication of application: **11.01.2023**
(21) Application number: 21779084.9
(22) Date of filing: 30.03.2021
(51) Int. Cl.: C07K 16/26, G01N 33/74

(54) **MONOCLONAL ANTIBODY AND POLYCLONAL ANTIBODY AND OXYTOCIN QUANTIFICATION METHOD BASED ON SAME**

(30) Priority: 01.04.2020 ES 202030265
(71) Applicant: Universidad de Murcia, 30100 Murcia (ES)
(72) Inventor: CERÓN MADRIGAL, José Joaquín, 30100 Murcia (ES); MARTÍNEZ SUBIELA, Silvia, 30100 Murcia (ES); LÓPEZ ARJONA, Marina, 30100 Murcia (ES)
(74) Representative: Manuel Illescas y Asociados, S.L.
(86) International application number: PCT/ES2021/070221
(87) International publication number: WO 2021/198548

(57) **Abstract**

The present invention relates to an *in vitro* method for the quantification of free oxytocin, protein-bound oxytocin that can be released in a reduction/alkylation treatment, and protein-bound oxytocin, in a sample, without performing a reduction/alkylation treatment, wherein said method comprising carrying out a first assay using a monoclonal antibody that binds specifically to free oxytocin and to protein-bound oxytocin that can be released in a reduction/alkylation treatment, and carrying out a second assay using a polyclonal antibody that binds specifically to protein-bound oxytocin. The invention also relates to the monoclonal antibody and polyclonal antibody, and to a composition and kit comprising same.

## Description

### TECHNICAL FIELD OF THE INVENTION

The present invention relates to immunological assays for the detection of hormones in biological material. In particular, the present invention relates to immunological assays involving monoclonal and/or polyclonal antibodies and in which oxytocin is detected in biological material.

### BACKGROUND OF THE INVENTION

Oxytocin is a hormone that is produced in the supraoptic and paraventricular nuclei of the hypothalamus and released into the circulatory system. Oxytocin is a hormone associated with the regulation of reproductive physiology events, such as childbirth and breastfeeding. In recent years, however, special importance has been given to its relationship with other different fields such as psychology and behaviour in both females and males. Oxytocin induces a decrease in cortisol levels, which reduces stress in both humans and animals. In general, oxytocin levels are measured in animals and humans to detect increases in plasma concentration indicative of a welfare and stress reduction situation. It is important to measure oxytocin levels to detect stressful states, which are harmful to health. Stressful states cause, among other effects, a reduction in the function of the immune system and predisposition to diseases. This deterioration of animal health and welfare is important in raised or farmed animals whose meat or other products derived therefrom, such as milk, will be used. The health and animal welfare of these livestock, in particular of pigs, becomes particularly important during the pregnancies of the females and after childbirth, affecting, for example, the care and survival of the offspring themselves.

Generally, the concentration of oxytocin has been measured by procedures that include an extraction or concentration of the sample by lyophilization. Oxytocin values in biological samples are generally very low. Both the extraction and the concentration of the sample aim to achieve sufficiently high values of oxytocin so that they can be measured.

The methods described in the prior art do not allow measuring all the oxytocin bound to proteins and, therefore, it is necessary in such methods to break the bonds between oxytocin and proteins. Reduction/alkylation (R/A) treatments are generally used for this purpose.

The application of R/A treatments to plasma samples results in oxytocin values several orders higher compared to the values in samples to which such treatments have not been applied (Brandtzaeg et al., 2016).

The methods used for extraction, lyophilization sample concentration, and reduction/alkylation treatments are technically complex and require several hours to perform, not being routinely used in analyses carried out on farm animals due to the long time they need. In addition, expensive reagents and laboratory materials are used in these processes, resulting in high analysis prices.

MacLean et al. measured oxytocin in dog saliva by various techniques, such as high performance liquid chromatography coupled to mass spectrometry (HPLC-MS) and Cayman Chemical and Arbor Assays commercial enzyme-linked immunoabsorption assays (ELISA), which do not use reduction and alkylation treatments (MacLean et al., 2018). This document, however, does not describe any assay in which free oxytocin and protein-bound oxytocin can be specifically quantified, nor does it describe the use of antibodies with such specificity against free oxytocin and protein-bound oxytocin.

Recently, a method for measuring oxytocin in sow saliva samples, at different times after delivery, in which oxytocin concentration was measured, was described with an AlphaLISA^{®} assay using monoclonal antibody-coated spheres (Lopez-Arjona et al., 2020). This document does not describe any assays in which free oxytocin and protein-bound oxytocin can be specifically quantified. It also does not describe the use of antibodies with specificity against free oxytocin and protein-bound oxytocin.

There is a need in the art to develop a method for measuring both free oxytocin and protein-bound oxytocin.

### DESCRIPTION OF THE INVENTION

In the present specification, the oxytocin is from mammals. Preferably, said mammals are selected from the group consisting of pigs, dogs, equines (comprising horses, mules and donkeys) and humans. More preferably, the oxytocin consists of the sequence SEQ ID NO: 1.

Herein, the term "sample" refers to a sample of a body fluid; to a sample of cells; to a sample of a tissue, or of an organ; or to a wash/rinse fluid sample obtained from an external or internal body surface. Samples can be obtained by well-known techniques. More preferably, the samples are samples of body fluids, e.g., preferably, blood, plasma, serum, urine, saliva, tears, and fluids obtainable from the mammary glands, e.g., milk. More preferably, the samples of body fluid are free of cells from the animal or subject. Tissue or organ samples may be obtained from any tissue or organ by, for example, a biopsy. Cells may be obtained or separated from body fluids or tissues or organs by techniques such as filtration, centrifugation, or cell sorting. Preferably, the samples of cells, tissues or organs are obtained from body fluids, cells, tissues or organs known or suspected to contain oxytocin. More preferably, the samples are samples of serum, plasma, saliva, urine, and cerebrospinal fluid.

Saliva has advantages compared to other types of sample, as it can be obtained in a simple way, without producing stress or pain. For this reason, saliva samples are increasingly used for the determination of biomarkers of stress or well-being in animals, preferably in pigs.

In the present patent, "free oxytocin" refers to what a person skilled in the art would understand by said term, which consists of oxytocin that is in free form, i.e., not bound to proteins, under physiological conditions.

An exemplary antibody is composed of two pairs of polypeptide chains. Each pair of polypeptide chains has a light chain (about 25 kD) and a heavy chain (about 50-70 kD). The N-terminal domain of each chain defines a variable region of about 100 to 110 or more amino acids responsible for antigen recognition. The light chain variable domain is also referred to as the "variable light chain" or "VL" and the heavy chain variable domain is also referred to as the "variable heavy chain" or "VH".

Herein, "CDR" or "complementarity determining region" refers to an amino acid sequence found in the variable domains of antibodies that gives antibodies their specificity for the antigen.

Herein, the term "ELISA" refers to enzyme-linked immunosorbent assay, which is an immunoassay technique in which an immobilized antigen is detected by an antibody bound to an enzyme (peroxidase, alkaline phosphatase, etc.,) capable of generating a detectable product from a substrate by a colour change or some other type of change caused by the enzymatic action on said substrate. In said technique, there may be a primary antibody that recognizes the antigen and that in turn is recognized by a secondary antibody bound to said enzyme. The antigen can be detected indirectly in the sample by colour changes measured by spectrophotometry.

The present invention provides an *in vitro* method for the quantification of free oxytocin, protein-bound oxytocin able to be released in a reduction/alkylation treatment, and protein-bound oxytocin, in a sample, without carrying out a reduction/alkylation treatment, wherein said method comprises:
(a) carrying out a first assay comprising:
   - adding to said sample a monoclonal antibody that specifically binds to free oxytocin and protein-bound oxytocin able to be released in a reduction/alkylation treatment, wherein said monoclonal antibody comprises:
      - a heavy chain CDR1 region consisting of the sequence SEQ ID NO: 6;
      - a heavy chain CDR2 region consisting of the sequence SEQ ID NO: 8.
      - a heavy chain CDR3 region consisting of the sequence SEQ ID NO: 10;
      - a light chain CDR1 region consisting of the sequence SEQ ID NO: 16;
      - a light chain CDR2 region consisting of the sequence SEQ ID NO: 18; and
      - a light chain CDR3 region consisting of the sequence SEQ ID NO: 20;
   - determining the amount or concentration of monoclonal antibody bound to free oxytocin and protein-bound oxytocin able to be released in a reduction/alkylation treatment; and
   - determining the amount or concentration of free oxytocin and protein-bound oxytocin able to be released in a reduction/alkylation treatment from the amount or concentration of monoclonal antibody bound to free oxytocin and to protein-bound oxytocin able to be released in a reduction/alkylation treatment;
(b) carrying out a second test, simultaneously or sequentially, in any order, comprising:
   - adding to said sample a polyclonal antibody that specifically binds to protein-bound oxytocin;
   - determining the amount or concentration of polyclonal antibody bound to protein-bound oxytocin; and
   - determining the amount or concentration of protein-bound oxytocin from the amount or concentration of polyclonal antibody bound to protein-bound oxytocin.

In a preferred embodiment of the method of the invention, said protein-bound oxytocin is albumin-bound oxytocin.

In a preferred embodiment, the method of the invention further comprises:
(c) adding the free oxytocin amount or concentration value obtained in step (a) to the protein-bound oxytocin amount or concentration value obtained in step (b).

In another preferred embodiment, the method of the invention further comprises:
(c) carrying out a mathematical operation between the free oxytocin amount or concentration value obtained in step (a) and the protein-bound oxytocin amount or concentration value obtained in step (b), such as division or multiplication.

The method of the invention avoids having to carry out a reduction/alkylation (R/A) procedure to determine the free oxytocin, this being one of the main technical differences with respect to the state of the art, which makes the quantification of oxytocin in the method of the invention cheaper and faster than other procedures. Accordingly, the method of the invention differs from the methods described in the prior art in that it can be done in less time and with less material means. The method of the invention, in addition to being able to be implemented in any laboratory, is especially indicated to be carried out in an ambulatory mode, *in situ,* in the animal farms.

The monoclonal antibody and the polyclonal antibody have different specificities. Such differences in specificity result from the different method of producing such antibodies. Oxytocin-KLH has been used for immunizations and oxytocin-BSA has been used to purify the monoclonal and polyclonal antibody. However, in the monoclonal antibody production method a cell fusion, a hybridoma, is used, while in the polyclonal antibody production procedure a direct inoculation of oxytocin-KLH in a rabbit is used, thereby causing the rabbit to produce antibodies. Therefore, due to differences in the production procedure, the antibodies have different specificities.

In one embodiment of the method of the invention, the variable domain of the heavy chain VH has at least 95%, 96%, 97%, 98%, or 99% identity to the sequence SEQ ID NO: 3 and the variable domain of the light chain VL has at least 95%, 96%, 97%, 98%, or 99% identity to the sequence SEQ ID NO: 13.

In one embodiment of the method of the invention, the variable domain of the heavy chain VH consists of the sequence SEQ ID NO: 3 and the variable domain of the light chain VL consists of the sequence SEQ ID NO: 13.

In one embodiment of the method of the invention, it is not necessary to subject the samples to any pre-processing.

If the samples are frozen, said samples are thawed at room temperature prior to application of the method of the invention.

In a preferred embodiment of the method of the invention, the monoclonal antibody and/or the polyclonal antibody are added at a concentration in the range 10-20 nM.

In another preferred embodiment of the method of the invention, the incubation time of the monoclonal antibody and/or the polyclonal antibody with the sample is between 1 and 2 hours.

In one embodiment of the method of the invention, the signal emitted by oxytocin from the sample is detected by binding to the monoclonal antibody and/or the polyclonal antibody. Such a signal may be an absorbance or fluorescence signal and may be detected by methods known in the state of the art, for example, in absorbance or fluorescence readers. The signal emitted by each antibody may be the same or different. The amount or concentration of oxytocin is determined, by methods known in the state of the art, from the value of said signal.

The method of the invention offers the following advantages:
(a) It allows to determine different types of oxytocin (free oxytocin, protein-bound oxytocin able to be released in a reduction/alkylation treatment and protein-bound oxytocin), without subjecting the sample to any R/A treatment prior to measurement, whereas in traditional methods it is necessary to subject the sample to a R/A treatment prior to measurement.
(b) It manages to measure, using the same analytical system, different types of oxytocin simultaneously or serially and in any order (first the free oxytocin and the protein-bound oxytocin able to be released in a reduction/alkylation treatment and, then, the protein-bound oxytocin, or vice versa, in the opposite order, first the protein-bound oxytocin and, then, the free oxytocin and the protein-bound oxytocin able to be released in a reduction/alkylation treatment), which makes possible important savings of time and also of materials, therefore being more economical from a commercial point of view. Simultaneous measurement can be carried out by binding each antibody to a different marker and using the same steps described above.

The method of the invention has been functionally tested by carrying out analytical validation tests. The method of the invention presents an imprecision, intra-assay and inter-assay, of less than 15%, is linear, presents percentages of oxytocin recovery between 80 and 120%, and the limit of detection and quantification allows measuring the concentrations of free oxytocin and protein-bound oxytocin that are normally present in biological samples. The correlation of the oxytocin values quantified with the assay with the monoclonal antibody of the invention and those quantified with the commercial ELISA kit of Cayman Chemical, which is used to measure free oxytocin with a pre-processing by R/A, was greater than 0.9, indicating that the values of the two methods have a high correlation.

In the method of the invention, the first and second assays (with the monoclonal antibody and the polyclonal antibody, or vice versa) are selected from the group consisting of immunofluorescence, immunohistochemistry, immunochromatography, luminescence, homogeneous amplified luminescent proximity assay, immunoelectrotransfer, ELISA, western blot, nephelometry, immunoturbidimetry, lateral chromatography, and microarrays.

In the method of the invention, the first and second assays (with the monoclonal antibody and the polyclonal antibody, or vice versa) are carried out simultaneously or sequentially.

In the method of the invention, the first and second assays (with the monoclonal antibody and the polyclonal antibody, or vice versa) are carried out in any order.

In the method of the invention, said monoclonal antibody and/or said polyclonal antibody are attached to a labelling probe or to a sphere attached to a labelling probe. Said labelling probes may be the same or different for each monoclonal or polyclonal antibody.

In the method of the invention, said labelling probe is selected from the group consisting of a fluorophore, a chromophore, a bioluminescent probe, a radioactive probe, an enzyme and colloidal gold.

In the method of the invention, the sample used to measure its total oxytocin content is selected from the group consisting of serum, plasma, saliva, urine, and cerebrospinal fluid.

The present invention also provides a monoclonal antibody that specifically binds to free oxytocin and protein-bound oxytocin able to be released in a reduction/alkylation treatment, wherein said monoclonal antibody comprises:
- a heavy chain CDR1 region consisting of the sequence SEQ ID NO: 6;
- a heavy chain CDR2 region consisting of the sequence SEQ ID NO: 8;
- a heavy chain CDR3 region consisting of the sequence SEQ ID NO: 10;
- a light chain CDR1 region consisting of the sequence SEQ ID NO: 16;
- a light chain CDR2 region consisting of the sequence SEQ ID NO: 18; and
- a light chain CDR3 region consisting of the sequence SEQ ID NO: 20.

In one embodiment of the monoclonal antibody of the invention, the variable domain of the heavy chain VH has at least 95% identity to the sequence SEQ ID NO: 3 and the variable domain of the light chain VL has at least 95% identity to the sequence SEQ ID NO: 13.

In one embodiment of the monoclonal antibody of the invention, the variable domain of the heavy chain VH consists of the sequence SEQ ID NO: 3 and the variable domain of the light chain VL consists of the sequence SEQ ID NO: 13.

The present invention also provides a polynucleotide encoding the monoclonal antibody of the invention, comprising the sequence SEQ ID NO: 22, which encodes the variable domain of the heavy chain, and sequence SEQ ID NO: 23, which encodes the variable domain of the light chain.

The present invention also provides the use of a polynucleotide that comprises the sequence SEQ ID NO: 22, which encodes the variable domain of the heavy chain, and sequence SEQ ID NO: 23, which encodes the variable domain of the light chain, in the production of the monoclonal antibody of the invention.

The present invention also provides a polyclonal antibody that specifically binds to protein-bound oxytocin.

The monoclonal antibody and/or polyclonal antibody of the invention are attached to a labelling probe or to a sphere attached to a labelling probe.

Said labelling probe attached to the monoclonal antibody and/or polyclonal antibody of the invention is selected from the group consisting of a fluorophore, a chromophore, a bioluminescent probe, a radioactive probe, an enzyme and colloidal gold. Said labelling probes may be the same or different for each monoclonal or polyclonal antibody.

The present invention also provides a composition comprising the monoclonal antibody and/or the polyclonal antibody of the invention.

The present invention also provides the use of such a composition comprising the monoclonal antibody of the invention and/or the polyclonal antibody of the invention, in the *in vitro* detection of free oxytocin and protein-bound oxytocin able to be released in a reduction/alkylation treatment and/or of protein-bound oxytocin in a sample.

The present invention also provides a kit for detecting free oxytocin and protein-bound oxytocin able to be released in a reduction/alkylation treatment and/or of protein-bound oxytocin in a sample, wherein said kit comprises:
(i) the monoclonal antibody and/or the polyclonal antibody or the composition of the invention comprising each of the monoclonal and polyclonal antibodies, or the combination of both; and
(ii) buffer solutions to carry out a reaction to form a complex between the monoclonal antibody and free oxytocin and protein-bound oxytocin able to be released in a reduction/alkylation treatment and/or between the polyclonal antibody and protein-bound oxytocin.

In the kit of the invention, said monoclonal antibody and/or polyclonal antibody are attached to a labelling probe or to a sphere attached to a labelling probe.

In the kit of the invention, said labelling probe is selected from the group consisting of a fluorophore, a chromophore, a bioluminescent probe, a radioactive probe, an enzyme and colloidal gold. Said labelling probes may be the same or different for each monoclonal or polyclonal antibody.

The kit of the invention further comprises reagents for carrying out the reaction to form such complexes between the monoclonal antibody and the free oxytocin and/or the polyclonal antibody and the protein-bound oxytocin and for carrying out the detection of such complexes.

The kit may integrate in a single device both antibodies, monoclonal and polyclonal, or the compositions containing each antibody, monoclonal and polyclonal, or comprise separate devices for each antibody or for each composition containing each antibody, to carry out each assay (a) or (b) of the method of the invention and both separate devices to be presented together in a single package or presentation, or to be presented separately, each in its own package or presentation.

The kit of the invention is used on a sample selected from the group consisting of serum, plasma, saliva, urine, and cerebrospinal fluid.

The kit of the invention can be used in any biological sample that may have oxytocin.

Additionally, the present invention provides the use of the monoclonal antibody of the invention in the *in vitro* detection in a sample of free oxytocin and protein-bound oxytocin able to be released in a reduction/alkylation treatment.

Preferably, said sample in which the monoclonal antibody of the invention is used contains free oxytocin and protein-bound oxytocin.

Additionally, the present invention also provides the use of the polyclonal antibody of the invention in the *in vitro* detection of protein-bound oxytocin in a sample.

Preferably, said sample in which the polyclonal antibody of the invention is used contains free oxytocin and protein-bound oxytocin.

In a preferred embodiment, said monoclonal antibody and/or said polyclonal antibody are used attached to a labelling probe.

Said labelling probe is selected from the group consisting of a fluorophore, a chromophore, a bioluminescent probe, a radioactive probe, an enzyme and colloidal gold. The labelling probes may be the same or different for each antibody, monoclonal or polyclonal.

One embodiment of the invention relates to the use of the kit of the invention in the *in vitro* detection, in a sample, of free oxytocin and protein-bound oxytocin able to be released in a reduction/alkylation treatment and/or protein-bound oxytocin.

### BRIEF DESCRIPTION OF THE FIGURES

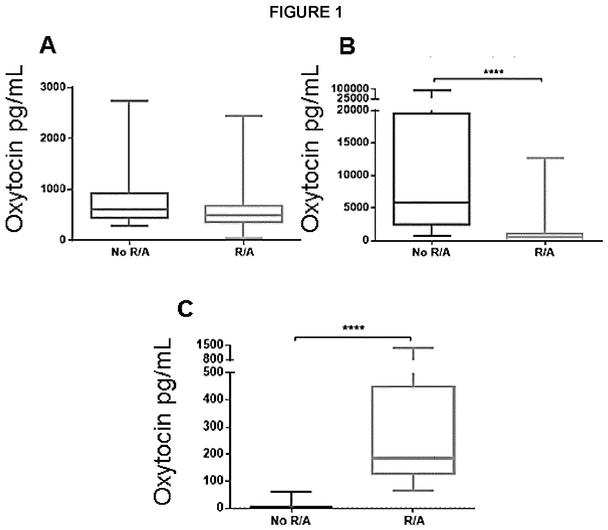

Figure 1. Results of the concentrations of oxytocin in the saliva of pigs before (No R/A) and after (R/A) treatment of the saliva samples with the AlphaLISA^{®} assay using the monoclonal antibody of the invention "P26C" (A), the AlphaLISA^{®} assay using the polyclonal antibody of the invention "PO-10" (B) and the assay with the ELISA kit of Cayman Chemical (C). The asterisks indicate significant differences (**** P ≤ 0.0001).

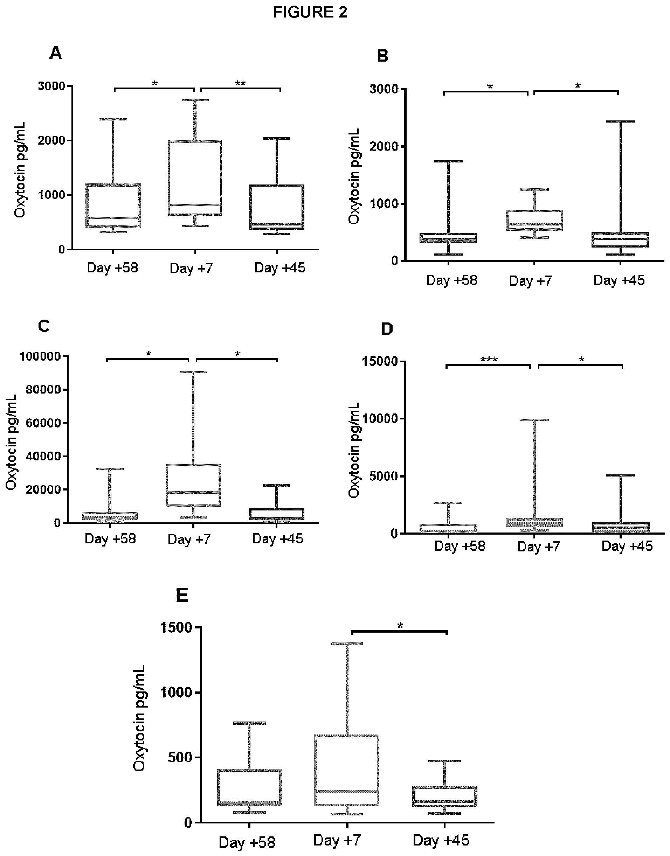

Figure 2. Changes in oxytocin concentrations on different days before and after farrowing, in sows: day 58 of gestation (Day +58), day 7 after farrowing (Day +7) and day 45 after farrowing (Day +45) with the AlphaLISA^{®} assay using the monoclonal antibody of the invention before (A) and after R/A treatment (B), the AlphaLISA^{®} assay using the polyclonal antibody of the invention before (C) and after R/A treatment (D) and the ELISA assay with the Cayman Chemical kit after R/A treatment (E). The asterisks indicate significant differences (*** P ≤ 0.001 ** P ≤ 0.01; * P ≤ 0.05).

### DESCRIPTION OF EMBODIMENTS

### Materials and methods

### Animals and samples

The samples used in this study were obtained from 10 female pigs *(Sus scrofa domesticus),* Large White. The pigs had *ad libitum* access to a balanced diet and water, with a temperature between 19-23°C and samples were taken between 8:30 and 10:00 h. A sample of each pig was taken 3 times during the study: day 58 of gestation, day 7 after farrowing (during lactation) and day 45 after farrowing. The animals were housed in the experimental farm of the University of Murcia (Murcia, Spain), in groups with a minimum space of 1.64 m² per animal on day 58 of gestation and individually on days 7 and 45 after delivery (recommendations described in Directive 2001/88/EC, 2001 and Directive 2001/93/EC, 2001).

Saliva samples were collected using Salivette tubes (Sarstedt, Aktiengesellschaft & Co.) containing a sponge. The pigs were allowed to chew the sponge, which was attached to a thin, flexible metal rod, until it was completely wet (or at least 1 minute). The sponges were then placed into the Salivette tubes which were immediately centrifuged at 3000 g for 10 minutes at 4°C. The saliva was then transferred into 1.5 mL tubes and stored at -80°C until analysis. In all cases, at least 500 microliters of saliva were obtained.

All procedures were approved by the Ethics Committee of the University of Murcia.

### Reduction/Alkylation Procedure (R/A)

The R/A procedure was carried out following the protocol described in Brandtzaeg et al. (2016). 30 samples of pig saliva were treated. 100 µl of each sample was diluted in 200 µl 50 mM Tris-HCI buffer (pH 8.0). Then, 5 µl of 0.5 M dithiothreitol (DTT) (GE Healthcare Life Sciences) was added to 100 µl of the diluted saliva sample, mixed for 30 seconds and incubated at 37°C for 45 minutes, followed by cooling to room temperature (22°C). Next, 15 µL of 0.5 M iodoacetamide (IAM) (GE Healthcare Life Sciences) was added to each solution, mixed for 30 seconds and incubated at 22°C in darkness for 20 minutes. Then, 80% acetonitrile (ACN) solution (Scharlab, SL), in ultrapure water (Millipore) (v/v), previously kept on ice, was added, mixed for 30 seconds and centrifuged for 15 minutes at 14,000 rpm in an Eppendorf Sorvall ST 8R centrifuge (Thermo Fisher Scientific). After centrifugation, the supernatant was pipetted into a new Eppendorf tube and evaporated to dryness in a Speed Vac concentrator (Eppendorf Concentrator 5301) followed by reconstitution in 100 µL saline phosphate buffer (PBS). The treated samples were diluted 1:2 with each buffer corresponding to each assay.

To assess whether the R/A procedure could affect the assays used in our study regardless of the effect it has on bound oxytocin, the assay buffer used for each assay was processed using the R/A protocol and analysed as an unknown sample in each of the three assays of our study.

### Example 1. Production of the monoclonal antibody of the invention "P26C" which specifically recognizes free oxytocin

### Generation of hybridoma

3 mice (BALB/c) were immunized with two intraperitoneal injections separated by an interval of 15 days with 50 µg of oxytocin-KLH (Cusabio) as antigen, where KLH is Keyhole Limpet Haemocyanin, that is, the haemocyanin of the keyhole limpet *(Megathura crenulata*). The antigen was emulsified with Freund's complete adjuvant (CCF, Sigma F-5881) at the first injection, and with Freund's incomplete adjuvant (CIF. Sigma F-5506) for the second intraperitoneal injection. The final immunization consisted of an intravenous or intra-splenic injection of 50 µg of antigen without the adjuvant.

Three days later, the spleen was extracted from the mice and the cells were fused to the Sp2/0-Ag14 murine myeloma cell line, developed from non-immunoglobulin-secreting BALB/c mice using 50% solution polyethylene glycol (Sigma). Hybridomas were selected by growth in RPMI 1640 medium (Biowhitaker) containing hypoxanthine, aminopterin, and thymidine (HAT), which was supplemented with 10% fetal bovine serum (Gibco), 2 mM glutamine (Gibco), 1 mM pyruvate (Sigma), and 1% penicillin/streptomycin (Gibco). In the HAT medium with aminopterin, only hybrid cells, consisting of B cells and myeloma cells, grow because B cells possess the entire panel of DNA synthesis rescue pathway enzymes and, therefore, can use the hypoxanthine and thymidine from the medium.

Finally, hybridoma supernatants were analysed by ELISA assay for the presence of anti-oxytocin immunoglobulin-secreting hybridomas. Positive hybridomas were cloned by limit dilution and expanded and frozen in liquid nitrogen.

### Identification of the monoclonal antibody

An analysis of the supernatants of the hybridomas generated by indirect ELISA assay using Oxytocin-BSA was carried out. To this end, they were incubated in 96-well flat-bottomed Nunc microplates (Maxisorp), with 50 µL of oxytocin-BSA (250 ng antigen per well) in carbonate-bicarbonate buffer pH 9.8 overnight, at 4°C, to allow the correct adhesion of the proteins to the plate. Subsequently the plates were washed with 100 µL 0.05% PBS/Tween 20 and blocked for 30 minutes at 37°C with 100 µL phosphate buffered saline (PBS) with 5% milk powder. The plates were then incubated 1 hour at 37°C, with 100 µL per well of each supernatant from the hybridomas. Next, three washes were carried out with 0.1% PBS/Tween 20 buffer. Then, 100 µL per well of a mouse anti-IgG antibody conjugated with horseradish peroxidase was added at a 1:2500 dilution and incubated for 1 hour at room temperature. After 5 washes with 0.1% PBS/Tween 20 buffer, to remove all labelled molecules not fixed in the form of immunocomplexes, the enzymatic substrate in solution (0.1M sodium citrate, 0.1M citric acid, 2,2'-azino-bis(3-ethylbenzothiazoline-6-sulfonic acid) (ABTS) and H₂O₂) was added. Subsequently, absorbance (OD) was measured on a spectrophotometer (BioTek) at a wavelength of 405 nm every 5 minutes to a total of 25 minutes.

The specific anti-oxytocin antibody was identified in the P26C hybridoma supernatant in said indirect ELISA assay using oxytocin-BSA. Clone P26C was selected because the highest absorbance signal was obtained with said clone in said ELISA assay, indicating a higher affinity for oxytocin than the other clones.

A dot-blot assay was carried out with clone P26C, which determined that it had reactivity against oxytocin of a high number of species, such as pig, dog, horse and human oxytocin.

### Purification of the monoclonal antibody P26C

For purification of the P26C monoclonal antibody from the supernatant obtained from the selected hybridoma, a HiTrap^{™} Protein G HP affinity column (GE Healthcare Life Sciences) was used using a ÄKTA pure chromatography system (GE Healthcare Life Sciences).

### Structural characterisation of the monoclonal antibody P26C

Total RNA was extracted from hybridoma cells and the cDNA was subsequently synthesized. The monoclonal antibody variable domains were then amplified by isotype-specific PCR, subcloned into a separate standard cloning vector, and sequenced. The isotype of the monoclonal antibody was determined to be IgGκ. The variable domains of 5 clones were sequenced and the sequence was found to be identical in all 5 clones. Table 1 shows the sequence details of monoclonal antibody P26C.

**Table 1**

| Fragment/Region/Sequence | SEQ ID NO |
|---|---|
| Variable domain of the heavy chain VH including signal peptide | SEQ ID NO: 2 |
| Variable domain of the heavy chain VH | SEQ ID NO: 3 |
| Signal peptide of the heavy chain VH | SEQ ID NO: 4 |
| FR1 framework region of the heavy chain VH | SEQ ID NO: 5 |
| CDR1 region of the heavy chain VH | SEQ ID NO: 6 |
| FR2 framework region of the heavy chain VH | SEQ ID NO: 7 |
| CDR2 region of the heavy chain VH | SEQ ID NO: 8 |
| FR3 framework region of the heavy chain VH | SEQ ID NO: 9 |
| CDR3 region of the heavy chain VH | SEQ ID NO: 10 |
| FR4 framework region of the heavy chain VH | SEQ ID NO: 11 |
| Variable domain of the light chain VL including signal peptide | SEQ ID NO: 12 |
| Variable domain of the light chain VL | SEQ ID NO: 13 |
| Signal peptide of the light chain VL | SEQ ID NO: 14 |
| FR1 framework region of the light chain VL | SEQ ID NO: 15 |
| CDR1 region of the light chain VL | SEQ ID NO: 16 |
| FR2 framework region of the light chain VL | SEQ ID NO: 17 |
| CDR2 region of the light chain VL | SEQ ID NO: 18 |
| FR3 framework region of the light chain VL | SEQ ID NO: 19 |
| CDR3 region of the light chain VL | SEQ ID NO: 20 |
| FR4 framework region of the light chain VL | SEQ ID NO: 21 |
| Nucleotide sequence encoding the variable fragment of the heavy chain VH including signal peptide | SEQ ID NO: 22 |
| Nucleotide sequence encoding the variable fragment of the light chain VL including signal peptide | SEQ ID NO: 23 |

### Example 2. Determination of free oxytocin concentrations in saliva samples

The saliva samples described in Materials and Methods were thawed at room temperature and 10 µL of 1:2 sample was diluted in assay buffer (AlphaLISA^{®} Universal buffer, Perkin elmer, Inc.). An AlphaLISA^{®} assay (Perkin Elmer, Inc.) was carried out, in 96-well plates, with a reaction volume of 50 µL per well, using spheres coated with the monoclonal antibody P26C and oxytocin concentrations in the samples were determined.

### Example 3. Production of the polyclonal antibody of the invention "PO-10" that specifically recognizes protein-bound oxytocin

A New Zealand rabbit (female, 2.5 kg, 3 months of age) was immunized with an oxytocin-KLH (Cusabio) conjugate as antigen, using 150 µg mixed in Freund's complete adjuvant for the first injection and Freund's incomplete adjuvant for the second and subsequent injections. One week after each immunization, several blood samples were collected and an ELISA assay was carried out to evaluate antibody titre using oxytocin-BSA-coated plates and a horseradish peroxidase-labelled rabbit anti-IgG secondary antibody.

### Purification of polyclonal antibody

An N-hydroxysuccinimide (NHS)-activated HiTrap^{™} HP (GE Healthcare Life Sciences) affinity column was used, to which oxytocin-BSA (Cusabio) was coupled and 1 mL batches of rabbit oxytocin antiserum were passed through the column using a ÄKTA pure chromatography system (GE Healthcare Life Sciences).

### Example 4. Determination of protein-bound oxytocin concentrations in saliva samples

The saliva samples described in the Materials and Methods section were thawed at room temperature and 10 µL of sample was diluted in assay buffer (AlphaLISA^{®} immunoassay buffer, Perkin Elmer Inc.). An AlphaLISA^{®} (Perkin Elmer, Inc.) indirect competition assay was carried out on 96-well plates, with a final reaction volume of 50 µL per well, using G-protein coated beads (Perkin Elmer Inc.) and oxytocin concentrations in the samples were determined. Different concentrations of biotinylated oxytocin (0-6 nM) and the polyclonal antibody of the invention PO-10 (10-20 nM) were assayed. Different concentrations of donor beads (Perkin Elmer Inc.) and G-protein coated acceptor beads were used at concentrations between 20-40 µg/mL.

### Example 5. Measurement of oxytocin by the method of the invention. Comparative assay with Cayman Chemical ELISA kit

Imprecision was calculated as variations between assays and intra-assays and expressed as coefficients of variation (CV). Five replicates of each sample (low, medium and high saliva samples) were analysed at the same time, to determine the intra-assay precision of the method. Five aliquots of each saliva sample were stored in plastic vials at -80°C until analysis. On the day of analysis, samples were brought to room temperature prior to oxytocin measurement. These samples were measured in duplicate five times over five different days to determine accuracy between assays using freshly prepared calibration curves.

Accuracy was assessed by evaluating linearity after dilution and recovery experiments. For the linearity evaluation, two samples with high and medium oxytocin values were diluted in series. Dilutions were 1:4 to 1:128 and 1:2 to 1:64 with assay buffer (AlphaLISA^{®} immunoassay buffer, Perkin Elmer Inc.) for the high and medium value sample, respectively. For the recovery test, a pig saliva sample with a known oxytocin concentration with different amounts of oxytocin standard (120, 80, 40, 20 and 10 ng/L) was diluted (1:2). The percentages of oxytocin concentrations measured at the expected oxytocin concentrations were then calculated.

The limit of detection (LD) was calculated from 12 replicate oxytocin assay buffer determinations (AlphaLISA^{®} immunoassay buffer, Perkin Elmer Inc.) as mean value plus 3 standard deviations (SD). The Lower Limit of Quantification (LLOQ) was calculated based on the lowest oxytocin concentration that could be measured with 20% accuracy. For this, a saliva sample was serially diluted with assay buffer and analysed five times at the same time for each dilution.

A commercially available ELISA kit from Cayman Chemical was used for the measurement of oxytocin for comparative purposes within the validation.

### Statistical analysis

The data obtained from different days of gestation and lactation were evaluated to determine the normality of the distribution, using the Shapiro-Wilk test, which provides a non-parametric distribution. Results below the limit of detection were set at the limit of detection of each method. The values were logarithmically transformed and the paired t-test was used for the differences between before and after the R/A procedure within each method. A one-way repeated measures ANOVA was applied to the log-transformed values, followed by uncorrected Fisher's LSD to compare oxytocin values obtained at different times on day 58 before farrowing and days 7 and 45 after farrowing. Spearman's correlation coefficients were calculated to evaluate the correlation between the three methods before and after the R/A procedure (GraphPad prism 5.00 for Windows).

In the polyclonal antibody immunoassay, intra-assay coefficients of variation (CV) were in the range 4.36-11.37% and inter-assay coefficients of variation were in the range 8.52-13.38%. Dilution of saliva samples resulted in linear regression equations with a correlation coefficient ranging from 0.98 to 0.99. The beta coefficients were significantly non-zero and the run test showed no deviation from linearity. The recovery results obtained were between 80% and 116% and the limit of detection of the assay was 58.4 pg/mL.

In the assay with the Cayman Chemical commercial kit, the intra-assay coefficients of variation were in the range 11.34-15.72% and the inter-assay coefficients of variation were in the range 18.56-21.04%. Dilution of saliva samples resulted in linear regression equations with a correlation coefficient of 0.99. The beta coefficients were significantly different from zero and the run test showed no deviation from linearity and the limit of detection of the assay was 4.66 pg/mL.

### Effects of R/A treatment

The results obtained for the samples are shown in Figure 1. In the AlphaLISA^{®} assay with the monoclonal antibody, the oxytocin concentrations of the samples showed no significant differences (P=0.0634) between samples with R/A procedure (mean value of 702.9 pg/mL) and without R/A procedure (mean value of 882.1 pg/mL). In the AlphaLISA^{®} assay with the polyclonal antibody, the oxytocin concentrations of the samples with the R/A procedure (mean value of 1308 pg/mL) were significantly lower (P<0.0001) than those obtained without the R/A procedure (mean value of 13492 pg/mL). Finally, in the assay with the Cayman Chemical commercial kit, oxytocin concentrations (P<0.0001) were significantly higher in samples with R/A procedure (mean value of 320.4 pg/mL) compared to those without R/A procedure (mean value of 11.8 pg/mL). Only 6 of a total of 30 samples without R/A procedure give results above the detection limit of this method and in samples below the detection limit the assay detection limit value was used for the statistical study.

When the buffers were analysed, the samples gave a result below the limit of detection before and after the R/A procedure in all three assays.

### Biological response of each assay

The biological response of oxytocin concentrations with each assay is shown in Figure 2.

In the AlphaLISA^{®} assay with the monoclonal antibody without R/A procedure, salivary oxytocin concentrations increased significantly on day 7 after farrowing, with a mean value of 1153 pg/mL, compared to gestation day 58 (mean value of 862 pg/mL) (1.34-fold; P=0.038) and day 45 after farrowing (mean value of 763 pg/mL) (1.51-fold; P=0.0038). When this method was applied with the R/A procedure, salivary oxytocin concentration increased significantly on day 7 after farrowing, with a mean value of 701.4 pg/mL, compared to day 58 gestation (mean value of 524.1 pg/mL) (1.34-fold; P=0.0159) and day 45 after farrowing (mean value of 552.1 pg/mL) (1.27-fold; P=0.0138).

In the AlphaLISA^{®} assay with the polyclonal antibody without R/A procedure, salivary oxytocin concentrations increased significantly on day 7 after farrowing, with a mean value of 25695 pg/mL, compared to gestation day 58 (mean value of 6896 pg/mL) (3.72-fold; P=0.0007) and day 45 after farrowing (mean value of 5939 pg/mL) (4.33-fold; P=0.0001). When the R/A procedure was applied to this assay, salivary oxytocin concentrations increased significantly on day 7 after farrowing, with a mean value of 1677 pg/mL, compared to gestation day 58 (mean value of 534.8 pg/mL) (3.14-fold; P=0.0003) and day 45 after farrowing (mean value of 906.5 pg/mL) (1.85-fold; P=0.0157).

Finally, when the Cayman Chemical ELISA kit was used without the R/A procedure, only 6 of the analysed samples were above the detection limit, so it was not possible to apply these conditions to evaluate possible biological changes. In the ELISA assay with the Cayman Chemical kit, with R/A procedure, salivary oxytocin concentrations increased significantly on day 7 after farrowing, with a mean value of 448.6 pg/mL, compared to day 45 after farrowing (mean value of 208.8 pg/mL) (2.15-fold; P=0.039) but there were no significant differences with gestation day 58 (mean value of 275.5 pg/mL) (1.63-fold; P=0.1999).

The summary of correlations between procedures is shown in Table 2. The asterisks indicate the statistical significance (****P* ≤ 0.001; ***P* ≤ 0.01; * P ≤ 0.05).

**Table 2**

| | AlphaLISA monoclonal no R/A | AlphaLISA monoclonal R/A | AlphaLISA polyclonal no R/A | AlphaLISA polyclonal R/A | Cayman kit no R/A | Cayman kit R/A |
|---|---|---|---|---|---|---|
| AlphaLISA monoclonal no R/A | | 0.65*** | 0.63*** | 0.39* | 0.51*** | 0.67*** |
| AlphaLISA monoclonal R/A | | | 0.68*** | 0.58*** | 0.47** | 0.53*** |
| AlphaLISA polyclonal no R/A | | | | 0.48** | 0.25 | 0.44** |
| AlphaLISA polyclonal R/A | | | | | 0.28 | 0.34 |
| Kit Cayman no R/A | | | | | | 0.36* |

When the R/A procedure was not carried out on the samples, the AlphaLISA^{®} assay with the monoclonal antibody showed a significant moderate correlation with both the AlphaLISA^{®} assay with the polyclonal antibody (P <0.001; r = 0.63) and the Cayman Chemical ELISA kit assay (P <0.001; r = 0.51). No significant correlation was found between the AlphaLISA^{®} assay with the polyclonal antibody and the Cayman Chemical ELISA kit assay. When the R/A procedure was carried out, the AlphaLISA^{®} assay with the monoclonal antibody showed a significant moderate correlation with both the AlphaLISA^{®} assay with the polyclonal antibody (P <0.001; r = 0.58) and the Cayman Chemical ELISA kit assay (P <0.001; r = 0.53). No significant correlation was found between the AlphaLISA^{®} assay with the polyclonal antibody and the Cayman Chemical ELISA kit assay.

### REFERENCES

Brandtzaeg et al. (2016). Proteomics tools reveal startlingly high amounts of oxytocin in plasma and serum. Sci Rep, 6, 31693.
López-Arjona et al. (2020). Oxytocin in saliva of pigs: an assay for its measurement and changes after farrowing. Domest Anim Endocrinol, 70, 106384.
MacLean et al. (2018). Validation of salivary oxytocin and vasopressin as biomarkers in domestic dogs. J Neurosci Methods, 293, 67-76.

## Claims

1. An *in vitro* method for the quantification of free oxytocin, protein-bound oxytocin able to be released in a reduction/alkylation treatment and protein-bound oxytocin, in a sample, without carrying out a reduction/alkylation treatment, said method comprising:
(a) carrying out a first assay comprising:
- adding to said sample a monoclonal antibody that specifically binds to free oxytocin and protein-bound oxytocin able to be released in a reduction/alkylation treatment, wherein said monoclonal antibody comprises:
- a heavy chain CDR1 region consisting of the sequence SEQ ID NO: 6;
- a heavy chain CDR2 region consisting of the sequence SEQ ID NO: 8;
- a heavy chain CDR3 region consisting of the sequence SEQ ID NO: 10;
- a light chain CDR1 region consisting of the sequence SEQ ID NO: 16;
- a light chain CDR2 region consisting of the sequence SEQ ID NO: 18; and
- a light chain CDR3 region consisting of the sequence SEQ ID NO: 20;
- determining the amount or concentration of monoclonal antibody bound to free oxytocin and protein-bound oxytocin able to be released in a reduction/alkylation treatment; and
- determining the amount or concentration of free oxytocin and protein-bound oxytocin able to be released in a reduction/alkylation treatment from the amount or concentration of monoclonal antibody bound to said free oxytocin and to protein-bound oxytocin able to be released in a reduction/alkylation treatment;
and
(b) carrying out a second assay comprising:
- adding to said sample a polyclonal antibody that specifically binds to protein-bound oxytocin;
- determining the amount or concentration of polyclonal antibody bound to protein-bound oxytocin; and
- determining the amount or concentration of protein-bound oxytocin from the amount or concentration of polyclonal antibody bound to said protein-bound oxytocin.

2. The method according to claim 1, wherein the variable domain of the heavy chain VH has at least 95% identity to the sequence SEQ ID NO: 3 and the variable domain of the light chain VL has at least 95% identity to the sequence SEQ ID NO: 13.

3. The method according to claim 1 or 2, wherein the variable domain of the heavy chain VH consists of the sequence SEQ ID NO: 3 and the variable domain of the light chain VL consists of the sequence SEQ ID NO: 13.

4. The method of any one of claims 1-3, wherein the first and second assays are selected from the group consisting of immunofluorescence, immunohistochemistry, immunochromatography, luminescence, homogeneous amplified luminescent proximity assay, immunoelectrotransfer, ELISA, western blot, nephelometry, immunoturbidimetry, lateral chromatography, and microarrays.

5. The method according to any one of claims 1-4, wherein the first and second assays are carried out simultaneously or sequentially.

6. The method according to any one of claims 1-5, wherein the first and second assays are carried out in any order.

7. The method according to any one of claims 1-6, wherein said monoclonal antibody and/or said polyclonal antibody are attached to a labelling probe or to a sphere attached to a labelling probe.

8. The method according to any one of claims 1-7, wherein said labelling probe is selected from the group consisting of a fluorophore, a chromophore, a bioluminescent probe, a radioactive probe, an enzyme, and colloidal gold.

9. The method according to any one of claims 1-8, wherein the labelling probes may be the same or different, for each antibody, monoclonal or polyclonal.

10. The method according to any one of claims 1-9, wherein said sample is selected from the group consisting of serum, plasma, saliva, urine, and cerebrospinal fluid.

11. A monoclonal antibody that specifically binds to free oxytocin and protein-bound oxytocin able to be released in a reduction/alkylation treatment, wherein said monoclonal antibody comprises:
- a heavy chain CDR1 region consisting of the sequence SEQ ID NO: 6;
- a heavy chain CDR2 region consisting of the sequence SEQ ID NO: 8;
- a heavy chain CDR3 region consisting of the sequence SEQ ID NO: 10;
- a light chain CDR1 region consisting of the sequence SEQ ID NO: 16;
- a light chain CDR2 region consisting of the sequence SEQ ID NO: 18; and
- a light chain CDR3 region consisting of the sequence SEQ ID NO: 20.

12. The monoclonal antibody according to claim 11, wherein the variable domain of the heavy chain VH has at least 95% identity to the sequence SEQ ID NO: 3 and the variable domain of the light chain VL has at least 95% identity to the sequence SEQ ID NO: 13.

13. The monoclonal antibody according to claim 11 or 12, wherein the variable domain of the heavy chain VH consists of the sequence SEQ ID NO: 3 and the variable domain of the light chain VL consists of the sequence SEQ ID NO: 13.

14. A polynucleotide encoding the monoclonal antibody according to any one of claims 11-13, wherein said polynucleotide comprises the sequence SEQ ID NO: 22, which encodes the variable domain of the heavy chain, and sequence SEQ ID NO: 23, which encodes the variable domain of the light chain.

15. Use of a polynucleotide according to claim 14, in the production of a monoclonal antibody according to claims 11-13.

16. Use of the monoclonal antibody according to any one of claims 11-13, in the *in vitro* detection of free oxytocin and protein-bound oxytocin able to be released in a reduction/alkylation treatment, in a sample.

17. A polyclonal antibody that specifically binds to protein-bound oxytocin.

18. Use of the polyclonal antibody according to claim 17, in the *in vitro* detection of protein-bound oxytocin in a sample.

19. Use of the monoclonal antibody and/or the polyclonal antibody according to claims 16 or 18, wherein said monoclonal antibody and/or said polyclonal antibody are attached to a labelling probe; and said labelling probe is the same or different for each of the monoclonal and polyclonal antibodies.

20. Use of the monoclonal and/or polyclonal antibody according to claim 19, wherein said labelling probe is selected from the group consisting of a fluorophore, a chromophore, a bioluminescent probe, a radioactive probe, an enzyme, and colloidal gold.

21. A composition comprising the monoclonal antibody according to claims 11-13 and/or the polyclonal antibody according to claim 17.

22. Use of a composition according to claim 21, in the *in vitro* detection of free oxytocin and protein-bound oxytocin able to be released in a reduction/alkylation treatment and/or protein-bound oxytocin, in a sample.

23. A kit for detecting free oxytocin and protein-bound oxytocin able to be released in a reduction/alkylation treatment and/or protein-bound oxytocin, in a sample, wherein said kit comprises:
(i) the monoclonal antibody according to any one of claims 11-13 and/or the polyclonal antibody according to claim 17 or the composition according to claim 21, comprising one or the other antibody, monoclonal or polyclonal, or both in combination; and
(ii) buffer solutions to carry out the reactions to form a complex between the monoclonal antibody and the free oxytocin and the protein-bound oxytocin able to be released in a reduction/alkylation treatment and/or between the polyclonal antibody and the protein-bound oxytocin.

24. The kit according to claim 23, wherein said monoclonal antibody and/or the polyclonal antibody are attached to a labelling probe or to a sphere attached to a labelling probe.

25. The kit of claim 24, wherein said labelling probe is selected from the group consisting of a fluorophore, a chromophore, a bioluminescent probe, a radioactive probe, an enzyme, and colloidal gold; and said labelling probe is the same or different for each of the monoclonal and polyclonal antibodies.

26. The kit according to any one of claims 23-25, further comprising reagents for carrying out the reaction to form said complexes between the monoclonal antibody and the free oxytocin and/or the polyclonal antibody and the protein-bound oxytocin and for carrying out the detection of said complexes.

27. The kit according to any one of claims 23-26, which may integrate into a single device both monoclonal and polyclonal antibodies, or the compositions containing each monoclonal and polyclonal antibody, or comprise separate devices for each antibody or for each composition containing each antibody, the two separate devices presented together in a single package or presentation, or presented separately, each in its own package or presentation.

28. Use of the kit according to any one of claims 23-27, in the *in vitro* detection of free oxytocin and protein-bound oxytocin able to be released in a reduction/alkylation treatment and/or protein-bound oxytocin, in a sample.

29. Use of the kit according to claim 28, wherein said sample is selected from the group consisting of serum, plasma, saliva, urine, and cerebrospinal fluid.
